# EUROPÄISCHE PATENTSCHRIFT

(11) **EP 0 473 208 B1**
(45) Veröffentlichungstag und Bekanntmachung des Hinweises auf die Patenterteilung: **15.03.1995**
(21) Anmeldenummer: 91119403.3
(22) Anmeldetag: 18.12.1986
(51) Int. Cl.: C09K 19/34, C07D 213/55, C07D 213/30, C07D 213/16, C07D 213/26, C07D 213/57, C07D 241/12, C07D 241/14

(54) **Ein Pyrazin oder Pyridin-Ring enthaltende flüssigkristalline Verbindungen**
Liquid crystal compounds containing a pyrazine or pyridine ring
Composés cristaux liquides contenant un cycle pyrazine ou pyridine

(30) Priorität: 03.01.1986 DE 3600052
(43) Veröffentlichungstag der Anmeldung: 04.03.1992
(62) Teilanmeldung aus: 87900721.9
(73) Patentinhaber: MERCK PATENT GmbH, D-64271 Darmstadt (DE)
(72) Erfinder: Gray, George William, Prof., Cottingham HU16 4DY (GB); Toyne, Kenneth Johnson, Dr., Hull HU6 8QW (GB); Lacey, David, Dr., Hull HU8 9NW (GB); Burrow, Michael Peter, Clifton, Bristol BS8 4PW (GB); Eidenschink, Rudolf, Dr., W-6500 Mainz (DE); Wächtler, Andreas, Dr., W-6103 Griesheim (DE); Weber, Georg, W-6106 Erzhausen (DE)

(56) Entgegenhaltungen:
- GB-A- 2 161 808
- JP-A-60 149 564
- JP-A-60 199 882

## Beschreibung

Heterocyclische Verbindungen der Formel I 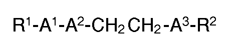 worin
R¹ und R² jeweils unabhängig voneinander eine Alkylgruppe mit 1-15 C-Atomen, worin auch eine oder zwei nicht benachbarte CH₂-Gruppen durch O-Atome und/oder -CO-Gruppen und/oder -O-CO-Gruppen und/oder -CO-O-Gruppen und/oder -O-COO-Gruppen und/oder -CHCN- und/oder -CH-Halogen-Gruppen ersetzt sein können, einer der Reste R¹ und R² auch F, Cl, Br oder CN,
A A² und A³ jeweils unabhängig voneinander eine unsubstituierte oder durch F- und/oder CI-Atome und/oder CH₃- und/oder CN-Gruppen ein- oder mehrfach substituierte 1,4-Cyclohexylengruppe, worin auch eine oder zwei nicht benachbarte CH₂-Gruppen durch O-Atome und/oder S-Atome ersetzt sein können, eine 1,4-Bicyclo-(2,2,2)-octylengruppe oder eine unsubstituierte oder durch F- und/oder CI-Atome und/oder CH₃- und/oder CN-Gruppen ein- oder mehrfach substituierte 1,4-Phenylengruppe, worin auch eine oder mehrere CH-Gruppen durch N ersetzt sein können,
   bedeutet, mit der Maßgabe , daß
   mindestens eine der Gruppen A¹ und A² eine Pyrazin-2,5-diylgruppe (Pa) oder eine Pyridin-2,5-diylgruppe (Py) ist.

Der Einfachheit halber bedeuten im folgenden Cy eine 1,4-Cyclohexylengruppe, Dio eine 1,3-Dioxan-2,5-diylgruppe, Dit eine 1,3-Dithian-2,5-diylgruppe, Bi eine 1,4-Bicyclo(2,2,2)-octylengruppe, Phe eine 1,4-Phenylengruppe (die gegebenenfalls auch lateral durch F, Cl, CH₃ oder CN substituiert sein kann), Pa eine Pyrazin-2,5-diylgruppe und Py eine Pyridin-2,5-diylgruppe.

Ähnliche Verbindungen sind z.B. aus DE-PS 25 35 046 bekannt. Die dort angegebenen Verbindungen enthalten im Gegensatz zu den vorliegenden keine Pyrazin-2,5-diyl- oder Pyridin-2,5-diylgruppe.

Ähnliche Pyridinverbindungen sind z. B. auch aus JP 60 149 564-A und JP 60 163 865-A bekannt. Ähnliche Pyrazinverbindungen sind beispielsweise in JP 60 199 882-A beschrieben.

Die Verbindungen der Formel I können wie ähnliche Verbindungen als Komponenten flüssigkristalliner Phasen verwendet werden, insbesondere für Displays, die auf dem Prinzip der verdrillten Zelle (TN-Displays), dem Guest-Host-Effekt, dem Effekt der Deformation aufgerichteter Phasen, dem Effekt der dynamischen Streuung oder dem SSFLC-Prinzip beruhen.

Der Erfindung lag die Aufgabe zugrunde, neue stabile flüssigkristalline oder mesogene Verbindungen aufzufinden, die als Komponenten flüssigkristalliner Phasen geeignet sind.

Es wurde gefunden, daß die Verbindungen der Formel I als Komponenten flüssigkristalliner Phasen vorzüglich geeignet sind. Insbesondere sind mit ihrer Hilfe stabile flüssigkristalline Phasen für TN-Displays mit hohen Multiplexraten, breiten Arbeitsbereichen und vorteilhaften elastischen Eigenschaften herstellbar. Weiterhin zeigen diese Phasen ausgeprägtes nematogenes Verhalten und günstige Schaltzeiten, insbesondere bei tiefen Temperaturen. Ferner eignen sich Verbindungen der Formel I, insbesondere optisch aktive Verbindungen der Formel I, als Komponenten ferroelektrischer Flüssigkristallphasen beispielsweise für Displays nach dem SSFLC-Prinzip.

Mit der Bereitstellung der Verbindungen der Formel 1 wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie oder andere Parameter eines solchen Dielektrikums zu optimieren. Die Verbindungen der Formel I eignen sich ferner als Zwischenprodukte zur Herstellung anderer Substanzen, die sich als Bestandteile flüssigkristalliner Phasen verwenden lassen.

Die Verbindungen der Formel 1 sind in reinem Zustand farblos und bilden flüssigkristalline Mesophasen in einem für die elektrooptische Verwendung günstig gelegenen Temperaturbereich. Chemisch, thermisch und gegen Licht sind sie sehr stabil.

Gegenstand der Erfindung sind somit die Verbindungen der Formel I sowie ein Verfahren zu ihrer Herstellung, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt,
oder daß man zur Herstellung von Estern der Formel I (worin R¹ und/oder R² eine Alkylgruppe bedeuten, worin eine oder zwei CH₂-Gruppen durch -O-CO-Gruppen und/oder -CO-O-Gruppen ersetzt sind)

eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von 1,3-Dioxanderivaten bzw. 1,3-Dithianderivaten der Formel I (worin A¹ oder A² oder A³ 1,3-Dioxan-2,5-diyl bzw. 1,3-Dithian-2,5-diyl bedeutet) einen entsprechenden Aldehyd mit einem entsprechenden Diol bzw. Dithiol umsetzt,
oder daß man zur Herstellung von Ethern der Formel I (worin R¹ und/oder R² eine Alkylgruppe bedeutet, worin eine oder zwei CH₂-Gruppen durch O-Atome ersetzt sind)

eine entsprechende Hydroxyverbindung verethert,
oder daß man zur Herstellung von Nitrilen der Formel I (worin R¹ oder R² CN bedeutet und/oder A¹ und/oder A² und/oder A³ durch mindestens eine CN-Gruppe substituiert sind) entsprechende Säureamide dehydratisiert oder die entsprechenden Säurehalogenide mit Sulfamid umsetzt,
oder daß man zur Herstellung von Verbindungen der Formel I (worin A¹ und/oder A² und/oder A³ durch F-und/oder CI-Atome substituiert sind) die entsprechenden Diazoniumsalze nach Schiemann bzw. Sandmeyer umsetzt,
und/oder daß man gegebenenfalls eine Base der Formel I durch Behandeln mit einer Säure in eines ihrer Säureadditionssalze umwandelt,
oder daß man gegebenenfalls eine Verbindung der Formel I aus einem ihrer Säureadditionssalze durch Behandeln mit einer Base freisetzt.

Weiterhin ist Gegenstand der Erfindung die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie Flüssigkristallanzeigeelemente, insbesondere elektrooptische Anzeigeelemente, die derartige Phasen enthalten.

Gegenstand der Erfindung ist auch ein ferroelektrisches Display nach Clark und Lagerwall, enthaltend Verbindungen der Formel I. Bevorzugt sind dabei Verbindungen der Formel I, in denen A¹, A², und A³ aromatische Ringe bedeuten. Insbesondere bevorzugt sind dabei Verbindungen, in denen mindestens ein aromatischer Ring lateral substituiert ist. R¹ und R² stellen dabei bevorzugt Alkyl- oder Alkoxygruppen dar, vorzugsweise mit jeweils 5 - 15 C-Atomen. R¹ und R² besitzen zusammen vorzugsweise mindestens 10 C-Atome.

Vor- und nachstehend haben R¹, R², A A² und A³

die angegebene Bedeutung, sofern nicht ausdrücklich etwas anderes vermerkt ist.

In den Verbindungen der vor- und nachstehenden Formeln bedeuten R¹ und R² vorzugsweise n-Alkyl, -O-n-Alkyl, -OCO-n-Alkyl, -COO-n-Alkyl oder -OCOO-n-Alkyl oder Oxaalkyl. In der n-Alkylgruppe kann auch eine CH₂-Gruppe durch -CH-CH₃-, -CH-Halogen oder -CH-CN ersetzt sein. Besonders bevorzugt sind Verbindungen der Formel I, worin eine der Gruppen R¹ und R² Alkyl und die andere Alkyl, Alkoxy oder Fluor, insbesondere bevorzugt Alkyl, bedeutet.

A¹ und A² sind bevorzugt Cy, Phe, Py oder Pa, ferner bevorzugt Dio. Bevorzugt enthält die Verbindung der Formel I nicht mehr als einen der Reste Dio, Dit oder Bi.

A³ ist bevorzugt Cy oder Phe, ferner Bi oder Dio. Insbesondere bevorzugt hat A³ die Bedeutung von lateral substituiertem 1,4-Phenylen.

Besonders bevorzugt sind Verbindungen der Formel I, worin eine der Gruppen A¹ und A² Py oder Pa, vorzugsweise Py, bedeutet und die anderen Gruppen A³, A¹ bzw. A² trans-1,4-Cyclohexylen oder 1,4-Phenylen bedeuten.

Sind A¹, A² oder A³ substituiert, so sind die Substituenten bevorzugt Cl- und/oder F-Atome, ferner auch CH₃- oder CN-Gruppen. Besonders bevorzugt sind dabei Verbindungen der Formel I, bei denen der laterale Substituent an einer 1,4-Phenylengrupp sitzt. Besonders bevorzugt ist Monofluorsubstitution an einer 1,4-Phenylengruppe.

Die Alkylreste in den Gruppen R¹ und/oder R² können geradkettig oder verzweigt sein. Vorzugsweise sind sie geradkettig, haben 2, 3, 4, 5, 6, 7, 8, 9, 10, 11 oder 12 C-Atome und bedeuten demnach bevorzugt Ethyl, Propyl, Butyl, Pentyl, Hexyl, Heptyl, Octyl, Nonyl, Decyl, Undecyl oder Dodecyl, ferner Methyl, Tridecyl, Tetradecyl oder Pentadecyl.

Falls R¹ und/oder R² Alkylreste bedeuten, in denen eine ("Alkoxy" bzw. "Oxaalkyl") oder zwei ("Alkoxyalkoxy" bzw. "Dioxaalkyl") CH₂-Gruppen durch O-Atome ersetzt sind, so können sie geradkettig oder verzweigt sein. Vorzugsweise sind sie geradkettig, haben 2, 3, 4, 5, 6 oder 7 C-Atome und bedeuten demnach bevorzugt Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, ferner Methoxy, Octoxy, Nonoxy, Decoxy, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl.

Verbindungen der Formel I sowie der vor- und nachstehenden Teilformeln mit verzweigten Flügelgruppen R¹ bzw. R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eine Kettenverzweigung. Bevorzugte verzweigte Reste R¹ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Octyloxy, 2-Chlorpropionyloxy, 2-Chlor-3-methylbutyryloxy, 2-Chlor-4-methylvaleryloxy, 2-Chlor-3-methylvaleryloxy, 2-Methyl-3-oxapentyl, 2-Methyl-3-oxa-hexyl. Optisch aktive Verbindungen der Formel I eignen sich als Komponenten chiral smektischer Flüssigkristallphasen und sind ferner auch in nematischen Materialien z.B. zur Vermeidung von reverse twist geeignet.

Besonders bevorzugte Pyrazinverbindungen sind solche, in denen eine der Gruppen A A² oder A³ eine lateral substituierte 1,4-Phenylengruppe ist. Insbesondere bevorzugt sind Pyrazine, die als A³ ein lateral substituiertes 1,4-Phenylen enthalten.

Bevorzugt sind auch Pyrazinverbindungen der Formel I, worin A¹ Pyrazin-2,5-diyl ist.

Bevorzugte Pyridinverbindungen enthalten mindestens eine 1,4-Cyclohexylengruppe oder eine lateral substituierte 1,4-Phenylengruppe.

Unter den Verbindungen der Formel I sowie der vor- und nachstehenden Teilformeln sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln 17, 18, 110, 126, 127, 145-148, 150, 151, 166 und 167, worin PheX 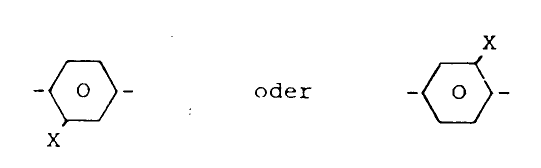 bedeutet.
X ist vorzugsweise F, Cl, CN oder CH₃, insbesondere F. 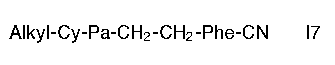 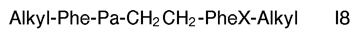 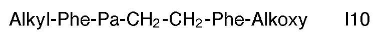 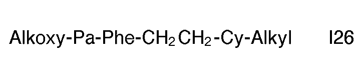 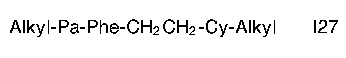 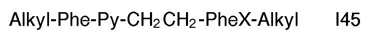 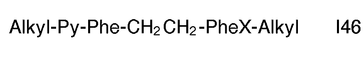 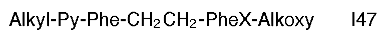 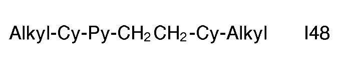 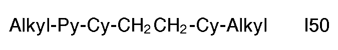 Alkoxy-Py-Cy-CH₂CH₂-Cy-Alkyl 151 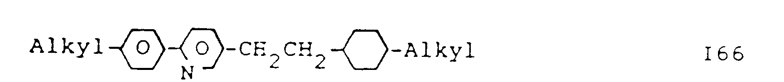 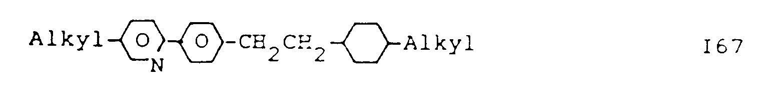 In den vorstehenden Formeln bedeutet Alkyl vorzugsweise eine Alkylgruppe mit 2 bis 10 C-Atomen und Alkoxy eine Alkoxygruppe mit 2 bis 12 C-Atomen. Weiterhin bevorzugt sind Verbindungen der vorstehenden Formeln, in denen an Stelle von Alkoxy eine 2-Oxaalkylgruppe mit 2 bis 12 C-Atomen vorhanden ist.

Unter den Verbindungen der Formel 1 sind diejenigen Stereoisomeren bevorzugt, in denen die gesättigten Ringe (z.B. Cy, Dio, Dit) trans-1,4-disubstituiert sind.

Diejenigen der vorstehend genannten Formeln, die eine oder mehrere der Gruppen Dio und/oder Dit enthalten, umschließen jeweils die beiden möglichen 2,5-Stellungsisomeren.

Die Verbindungen der Formel I werden nach an sich bekannten Methoden hergestellt, wie sie in der Literatur (z.B. in den Standardwerken wie Houben Weyl, Methoden der Organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht erwähnten Varianten Gebrauch machen.

Der Fachmann kann durch Routinemethoden entsprechende Synthesemethoden aus dem Stand der Technik entnehmen (z.B. DE-OS 23 44 732, 24 50 088, 24 29 093, 25 02 904, 26 36 684, 27 01 591 und 27 52 975 betreffend Verbindungen mit 1,4-Cyclohexylen und 1,4-Phenylen-Gruppen; DE-OS 29 44 905 und 32 27 916 betreffend Verbindungen mit 1,3-Dioxan-2,5-diyl-Gruppen; DD 160 061 betreffend Verbindungen mit 1,3-Dithian-2,5-diyl-Gruppen;
US 4,261,652 und 4,219,256 betreffend Verbindungen mit 1,4-Bicyclo(2,2,2)-octylen-Gruppen; und DE-OS 32 01 721 betreffend Verbindungen mit -CH₂CH₂-Brückengliedern.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion entsprechen der Formel I, können aber an Stelle eines Cyclohexanrings einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eines H-Atomes eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0 und etwa 200 sowie Drucken zwischen etwa 1 und 200 bar in einem inerten Lösungsmittel, z.B. einem Alkohol wie Methanol, Ethanol oder Isopropranol, einem Ether wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. Pt0₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120 _{°}C) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperaturen zwischen etwa 100 und 200 ° C) zu den entsprechenden Verbindungen der Formel 1, die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAIH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100 _{°}C. Doppelbindungen können (auch in Gegenwart von CN-Gruppen!) mit NaBH₄ oder Tributylzinnhydrid in Methanol hydriert werden.

Ester der Formel I (R¹ und/oder R² = Alkyl, worin eine oder zwei CH₂-Gruppen durch -O-CO- und/oder -CO-O-Gruppen ersetzt sind)

können auch durch Veresterung entsprechender Carbonsäure (oder ihren reaktionsfähigen Derivaten) mit Alkoholen bzw. Phenolen (oder ihrer reaktionsfähigen Derivaten) erhalten werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50 und + 250 _{°} , vorzugsweise zwischen -20 und + 80 ° . Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab. So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimatallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und zusammen mit Natriumhydrogencarbonat oder Kaliumcarbonat unter Rühren in Aceton oder Diethylether suspendiert und diese Suspension mit einer Lösung des Säurechlorids oder Anhydrids in Diethylether, Aceton oder DMF versetzt, zweckmäßig bei Temperaturen zwischen etwa -25 ° und + 20 ° .

Dioxanderivate bzw. Dithianderivate der Formel I (worin eine der Gruppen A¹ und/oder A² und/oder A³ eine 1,3-Dioxan-2,5-diyl-Gruppe bzw. eine 1,3-Dithian-2,5-diyl-Gruppe bedeutet) werden zweckmäßig durch Reaktion eines entsprechenden Aldehyds mit einem entsprechenden 1,3-Diol bzw. einem entsprechenden 1,3-Dithiol (oder einem seiner reaktionsfähigen Derivate) hergestellt, vorzugsweise in Gegenwart eines inerten Lösungsmittels wie Benzol oder Toluol und/oder eines Katalysators z.B. einer starken Säure wie Schwefelsäure, Benzol- oder p-Toluolsulfonsäure, bei Temperaturen zwischen etwa 20 und etwa 150 ° , vorzugsweise zwischen 80 und 120°. Als reaktionsfähige Derivate der Ausgangstoffe eignen sich in erster Linie Acetale.

Die genannten Aldehyde, 1,3-Diole und 1,3-Dithiole sowie ihre reaktionsfähigen Derivate sind zum Teil bekannt, zum Teil können sie ohne Schwierigkeiten nach Standardverfahren der organischen Chemie aus literaturbekannten Verbindungen hergestellt werden. Beispielsweise sind die Aldehyde durch Oxydation entsprechender Alkohole oder durch Reduktion entsprechender Carbonsäuren oder ihrer Derivate, die Diole durch Reduktion entsprechender Diester, sowie die Dithiole durch Umsetzung entsprechender Dihalogenide mit NaSH erhältlich.

Zur Herstellung von Nitrilen der Formel I (worin R¹ oder R² CN bedeutet) können entsprechende Säureamide dehydratisiert werden. Die Amide sind z.B. aus entsprechenden Estern oder Säurehalogeniden durch Umsetzung mit Ammoniak erhältlich. Als wasserabspaltende Mittel eignen sich beispielsweise anorganische Säurechloride wie SOCI₂, PCl₃, PCls, POCl₃, S0₂C1₂, COCI₂, ferner P₂O₅, P₂Ss, AlCl₃ (z.B. als Doppelverbindung mit NaCI), aromatische Sulfonsäuren und Sulfonsäurehalogenide. Man kann dabei in Gegenwart oder Abwesenheit eines inerten Lösungsmittels bei Temperaturen zwischen etwa 0 und 150 arbeiten; als Lösungsmittel kommen z.B. Basen wie Pyridin oder Triethylamin, aromatische Kohlenwasserstoffe wie Benzol, Toluol oder Xylol oder Amide wie DMF in Betracht.

Zur Herstellung der vorstehend genannten Nitrile der Formel 1 kann man auch entsprechende Säurehalogenide, vorzugsweise die Chloride, mit Sulfamid umsetzen, zweckmäßig in einem inerten Lösungsmittel wie Tetramethylsulfon bei Temperaturen zwischen etwa 80 und 150°, vorzugsweise bei 120°. Nach üblicher Aufarbeitung kann man direkt die Nitrile isolieren.

Ether der Formel I (worin R¹ und/oder R² eine Alkylgruppe bedeutet, worin eine oder zwei CH₂-Gruppen durch O-Atome ersetzt sind)

sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂C0₃ oder K₂C0₃ in das entsprechende Alkalimetallalkoholat oder Alkalimetallphenolat übergeführt wird. Dieses kann dann mit dem entsprechenden Alkylhalogenid, -sulfonat oder Dialkylsulfonat umgesetzt werden, zweckmäßig in einem inerten Lösungsmittel wie Aceton, 1,2-Dimethoxyethan, DMF oder Dimethylsulfoxid oder auch einen Überschuß an wäßriger oder wäßrig-alkoholischer NaOH oder KOH bei Temperaturen zwischen etwa 20 und 100 ° .

Zur Herstellung von Nitrilen der Formel I (worin R¹ oder R² CN bedeutet und/oder A¹ und/oder A² und/oder A³ durch mindestens eine CN-Gruppe substituiert ist) können auch entsprechend Chlor- oder Bromverbindungen der Formel I mit einem Cyanid umgesetzt werden, zweckmäßig mit einem Metallcyanid wie NaCN, KCN oder Cu₂(CN)₂, z.B. in Gegenwart von Pyridin in einem inerten Lösungsmittel wie DMF oder N-Methylpyrrolidon bei Temperaturen zwischen 20 _{°} und 200 ° .

Zur Herstellung von lateral substituierten Fluor- oder Chlor-Verbindungen der Formel I (worin A¹ und/oder A² und/oder A³ Aromaten bedeuten, R¹ und R² die üblichen Bedeutungen haben können) können entsprechende Aminoverbindungen (erhältlich durch Reduktion der entsprechenden Nitroverbindungen nach Standardmethoden, z.B. durch katalytische Hydrierung, durch Behandlung mit wäßrigem Dithionit oder mit Zinn-(II)-chlorid und Salzsäure) mit Natriumnitrit und entweder mit Tetrafluorborsäure (zur Einführung eines F-Atoms) oder mit Kupfer-(I)-chlorid (zur Einführung eines CI-Atoms) zu den Diazoniumsalzen umgesetzt werden, die dann bei Temperaturen von 100 ° -250 0 thermisch zersetzt werden.

Eine Base der Formel 1 kann mit einer Säure in das zugehörige Säureadditionssalz überführt werden. Für diese Umsetzung können anorganische Säuren verwendet werden, z.B. Schwefelsäure, Salpetersäure, Halogenwasserstoffsäure wie Chlorwasserstoffsäure oder Bromwasserstoffsäure, Phosphorsäuren wie Orthophosphorsäure, Sulfaminsäure, säure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und-disulfonsäuren, Laurylschwefelsäure.

Umgekehrt ist es möglich, aus einem Säureadditionssalz einer Verbindung der Formel I die Base der Formel I durch Behandeln mit einer Base freizusetzen, z.B. mit einer starken anorganischen Base wie KOH oder NaOH.

Die erfindungsgemäßen flüssigkristallinen Phasen bestehen aus 2 bis 15, vorzugsweise 3 bis 12 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl-oder Cyclohexylbenzoate, Cyclohexancarbonsäurephenyl- oder cyclohexylester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, Phenyl- oder Cyclohexyl-1,3-dithiane, 1-Phenyl-2-cyclohexylethane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel II charakterisieren, 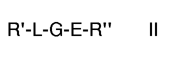 worin L und E je ein carbo- oder heterocyclisches Ringsystem aus der aus 1,4-disubstituierten Benzol und Cyclohexanringen, 4,4'-disubstituierten Biphenyl-, Phenylcyclohexan und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe, 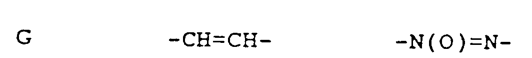 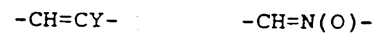 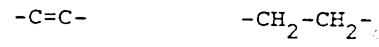 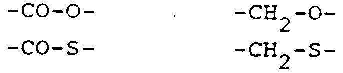 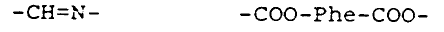 oder eine C-C-Einfachbindung, Y Halogen, vorzugsweise Chlor, oder CN, und R' und R" Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, N0₂, CH₃, F, CI oder Br bedeuten.

Bei den meisten dieser Verbindungen sind R' und R" voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich. Alle diese Substanzen sind nach literaturbekannten Methoden herstellbar.

Die erfindungsgemäßen Phasen enthalten etwa 0,1 bis 99, vorzugsweise 10 bis 95 %, einer oder mehrerer Verbindungen der Formel I.

Weiterhin bevorzugt sind erfindungsgemäße Dielektrika enthaltend 0,1 bis 40, vorzugsweise 0,5 bis 30 %, einer oder mehrerer Verbindungen der Formel I.

Die Herstellung der erfindungsgemäßen Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Dielektrika nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vgl. z.B. I. Haller et al., Mol.Cryst.Liq.Cryst. Band 24, Seiten 249-258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE-OS 22 09 127, 22 40 854, 23 21 632, 23 38 281, 24 50 088, 26 37 430, 28 53 728 und 29 02 177 beschrieben.

Die folgenden Beispiele sollen die Erfindung erläutern, ohne sie zu begrenzen. C, S, Ch, N und I bedeuten kristalliner, smektischer, cholesterischer, nematischer und isotroper Zustand. Die Zahlen dazwischen stellen die Übergangstemperturen dar. F. bedeutet Schmelzpunkt. Vor- und nachstehend bedeuten Prozentangaben Gewichtsprozent; alle Temperaturangaben sind in Grad Celsius angegeben. "Übliche Aufarbeitung" bedeutet: man gibt Wasser hinzu, extrahiert mit Methylenchlorid, trennt ab, trocknet die organische Phase, dampft ein und reinigt das Produkt durch Kristallisation und/oder Chromatographie.

### Beispiel 1

Zu einer Lösung von 0,019 mol 3-Chlor-1-[4-(trans-4-pentylcyclohexylethyl)phenyl]propan-1-on (F. 73-74_{°}, herstellbar durch a) Umsetzung von Benzol mit trans-4-Pentylcyclohexylacetylchlorid nach Friedel-Crafts b) anschließende Reduktion nach Huang-Minlon zu 1-(trans-4-Pentylcyclohexyl)-2-phenylethan(Siedepunkt: 125_{°}/0,3 mmHg) und c) Umsetzung dieser Verbindung mit 3-Chlorpropanoylchlorid in CH₂CI₂ und AlCl₃) in 5 ml Tetrahydrofuran gibt man unter Rühren 0,02 mol Triethylamin. Man rührt eine Stunde und gibt dann 0,019 mol 1-Piperidinopenten (Siedepunkt: 101-104_{°}/18 mmHg, herstellbar nach G. Stork et al., J. Amer. Chem. Soc. 1963, 85, 207 und C. Mannich und H. Davidsen, Chem. Ber. 1936, 69, 2106) in 5 ml THF zu und rührt 72 Stunden.

Das Lösungsmittel wird entfernt und der Rückstand in Ethanol-Wasser (1:1, 10 ml) aufgenommen und mit 0,059 mol Hydroxylaminhydrochlorid unter Rühren 72 Stunden lang erhitzt.

Das Lösungsmittel wird abgedampft und der Rückstand mit NaOH-Lösung (10 %, 100 ml) und Ether (50 ml) behandelt. Die organische Phase wird wie üblich aufgearbeitet und man erhält nach Reinigung durch Säulenchromatographie (neutrales Aluminiumoxid, Elutionsmittel Petrolether-Chloroform) und Umkristallisation 2-p-[2'-(trans-4-Pentylcyclohexyl)ethyl]phenyl-5-propylpyridin mit C 36 ° S_{G} 53 ° S_{B} 100° N 150 ° I (vgl. auch C. Botteghi et al., Synth. Comm. 1979, 9(2), 69).

Analog werden hergestellt:
2-p-[2'-(trans-4-Propylcyclohexyl)ethyl]phenyl-5-propylpyridin, C 81 ° N 149 ° I
2-p-[2'-(trans-4-Propylcyclohexyl)ethyl]phenyl-5-butylpyridin, C 88 ° N 142 ° I
2-p-[2'-(trans-4-Propylcyclohexyl)ethyl]phenyl-5-pentylpyridin, C 93 N 156 ° I
2-p-[2'-(trans-4-Propylcyclohexyl)ethyl]phenyl-5-hexylpyridin
2-p-[2'-(trans-4-Propylcyclohexyl)ethyl]phenyl-5-heptylpyridin
2-p-[2'-(trans-4-Propylcyclohexyl)ethyl]phenyl-5-octylpyridin
2-p-[2'-(trans-4-Propylcyclohexyl)ethyl]phenyl-5-ethylpyridin
2-p-[2'-(trans-4-Ethylcyclohexyl)ethyl]phenyl-5-propylpyridin
2-p-[2'-(trans-4-Ethylcyclohexyl)ethyl]phenyl-5-butylpyridin
2-p-[2'-(trans-4-Ethylcyclohexyl)ethyl]phenyl-5-pentylpyridin
2-p-[2'-(trans-4-Ethylcyclohexyl)ethyl]phenyl-5-hexylpyridin
2-p-[2'-(trans-4-Ethylcyclohexyl)ethyl]phenyl-5-heptylpyridin
2-p-[2'-(trans-4-Ethylcyclohexyl)ethyl]phenyl-5-octylpyridin
2-p-[2'-(trans-4-Ethylcyclohexyl)ethyl]phenyl-5-ethylpyridin
2-p-[2'-(trans-4-Butylcyclohexyl)ethyl]phenyl-5-propylpyridin
2-p-[2'-(trans-4-Butylcyclohexyl)ethyl]phenyl-5-butylpyridin
2-p-[2'-(trans-4-Butylcyclohexyl)ethyl]phenyl-5-pentylpyridin, C 102° N 137 ° I
2-p-[2'-(trans-4-Butylcyclohexyl)ethyl]phenyl-5-hexylpyridin
2-p-[2'-(trans-4-Butylcyclohexyl)ethyl]phenyl-5-heptylpyridin
2-p-[2'-(trans-4-Butylcyclohexyl)ethyl]phenyl-5-octylpyridin, C 79 ° S 148 ° N 153 ° I
2-p-[2'-(trans-4-Butylcyclohexyl)ethyl]phenyl-5-ethylpyridin
2-p-[2'-(trans-4-Pentylcyclohexyl)ethyl]phenyl-5-butylpyridin
2-p-[2'-(trans-4-Pentylcyclohexyl)ethyl]phenyl-5-pentylpyridin, C 99 ° S 141 ° N 146 ° I
2-p-[2'-(trans-4-Pentylcyclohexyl)ethyl]phenyl-5-hexylpyridin
2-p-[2'-(trans-4-Pentylcyclohexyl)ethyl]phenyl-5-heptylpyridin
2-p-[2'-(trans-4-Pentylcyclohexyl)ethyl]phenyl-5-octylpyridin
2-p-[2'-(trans-4-Pentylcyclohexyl)ethyl]phenyl-5-ethylpyridin
2-p-[2'-(trans-4-Hexylcyclohexyl)ethyl]phenyl-5-propylpyridin
2-p-[2'-(trans-4-Hexylcyclohexyl)ethyl]phenyl-5-butylpyridin
2-p-[2'-(trans-4-Hexylcyclohexyl)ethyl]phenyl-5-pentylpyridin
2-p-[2'-(trans-4-Hexylcyclohexyl)ethyl]phenyl-5-hexylpyridin
2-p-[2'-(trans-4-Hexylcyclohexyl)ethyl]phenyl-5-heptylpyridin
2-p-[2'-(trans-4-Hexylcyclohexyl)ethyl]phenyl-5-octylpyridin
2-p-[2'-(trans-4-Hexylcyclohexyl)ethyl]phenyl-5-ethylpyridin
2-p-[2'-(trans-4-Heptylcyclohexyl)ethyl]phenyl-5-propylpyridin
2-p-[2'-(trans-4-Heptylcyclohexyl)ethyl]phenyl-5-butylpyridin
2-p-[2'-(trans-4-Heptylcyclohexyl)ethyl]phenyl-5-pentylpyridin
2-p-[2'-(trans-4-Heptylcyclohexyl)ethyl]phenyl-5-hexylpyridin
2-p-[2'-(trans-4-Heptylcyclohexyl)ethyl]phenyl-5-heptylpyridin
2-p-[2'-(trans-4-Heptylcyclohexyl)ethyl]phenyl-5-octylpyridin
2-p-[2'-(trans-4-Heptylcyclohexyl)ethyl]phenyl-5-ethylpyridin
2-p-[2'-(trans-4-Octylcyclohexyl)ethyl]phenyl-5-propylpyridin
2-p-[2'-(trans-4-Octylcyclohexyl)ethyl]phenyl-5-butylpyridin
2-p-[2'-(trans-4-Octylcyclohexyl)ethyl]phenyl-5-pentylpyridin
2-p-[2'-(trans-4-Octylcyclohexyl)ethyl]phenyl-5-hexylpyridin
2-p-[2'-(trans-4-Octylcyclohexyl)ethyl]phenyl-5-heptylpyridin
2-p-[2'-(trans-4-Octylcyclohexyl)ethyl]phenyl-5-octylpyridin
2-p-[2'-(trans-4-Octylcyclohexyl)ethyl]phenyl-5-ethylpyridin
2-p-[2'-(trans-4-Propylcyclohexyl)ethyl]phenyl-5-ethoxypyridin, C 121 ° S 142° N 157 ° I
2-p-[2'-(trans-4-Butylcyclohexyl)ethyl]phenyl-5-ethoxypyridin
2-p-[2'-(trans-4-Pentylcyclohexyl)ethyl]phenyl-5-ethoxypyridin
2-p-[2'-(trans-4-Hexylcyclohexyl)ethyl]phenyl-5-ethoxypyridin
2-p-[2'-(trans-4-Heptylcyclohexyl)ethyl]phenyl-5-ethoxypyridin
2-p-[2'-(trans-4-Octylcyclohexyl)ethyl]phenyl-5-ethoxypyridinn
2-p-[2'-(trans-4-Propylcyclohexyl)ethyl]phenyl-5-propoxypyridin
2-p-[2'-(trans-4-Butylcyclohexyl)ethyl]phenyl-5-propoxypyridin
2-p-[2'-(trans-4-Pentylcyclohexyl)ethyl]phenyl-5-propoxypyridin
2-p-[2'-(trans-4-Hexylcyclohexyl)ethyl]phenyl-5-propoxypyridin
2-p-[2'-(trans-4-Heptylcyclohexyl)ethyl]phenyl-5-propoxypyridin
2-p-[2'-(trans-4-Octylcyclohexyl)ethyl]phenyl-5-propoxypyridin
2-p-[2'-(trans-4-Propylcyclohexyl)ethyl]phenyl-5-butoxypyridin, C 111 ° S 135° N 142 ° I
2-p-[2'-(trans-4-Butylcyclohexyl)ethyl]phenyl-5-butoxypyridin
2-p-[2'-(trans-4-Pentylcyclohexyl)ethyl]phenyl-5-butoxypyridin
2-p-[2'-(trans-4-Hexylcyclohexyl)ethyl]phenyl-5-butoxypyridin
2-p-[2'-(trans-4-Heptylcyclohexyl)ethyl]phenyl-5-butoxypyridin
2-p-[2'-(trans-4-Octylcyclohexyl)ethyl]phenyl-5-butoxypyridin
2-p-[2'-(trans-4-Propylcyclohexyl)ethyl]phenyl-5-pentoxypyridin
2-p-[2'-(trans-4-Butylcyclohexyl)ethyl]phenyl-5-pentoxypyridin
2-p-[2'-(trans-4-Pentylcyclohexyl)ethyl]phenyl-5-pentoxypyridin
2-p-[2'-(trans-4-Hexylcyclohexyl)ethyl]phenyl-5-pentoxypyridin
2-p-[2'-(trans-4-Heptylcyclohexyl)ethyl]phenyl-5-pentoxypyridin
2-p-[2'-(trans-4-Octylcyclohexyl)ethyl]phenyl-5-pentoxypyridin
2-p-[2'-(trans-4-Propylcyclohexyl)ethyl]phenyl-5-hexoxypyridin
2-p-[2'-(trans-4-Butylcyclohexyl)ethyl]phenyl-5-hexoxypyridin
2-p-[2'-(trans-4-Pentylcyclohexyl)ethyl]phenyl-5-hexoxypyridin
2-p-[2'-(trans-4-Hexylcyclohexyl)ethyl]phenyl-5-hexoxypyridin
2-p-[2'-(trans-4-Heptylcyclohexyl)ethyl]phenyl-5-hexoxypyridin
2-p-[2'-(trans-4-Octylcyclohexyl)ethyl]phenyl-5-hexoxypyridin
2-[2-Fluor-4-(2'-trans-4-ethylcyclohexylethyl)phenyl]-5-methylpyridin
2-[2-Fluor-4-(2'-trans-4-propylcyclohexylethyl)phenyl]-5-methylpyridin
2-[2-Fluor-4-(2'-trans-4-butylcyclohexylethyl)phenyl]-5-methylpyridin
2-[2-Fluor-4-(2'-trans-4-pentylcyclohexylethyl)phenyl]-5-methylpyridin
2-[2-Fluor-4-(2'-trans-4-hexylcyclohexylethyl)phenyl]-5-methylpyridin
2-[2-Fluor-4-(2'-trans-4-heptylcyclohexylethyl)phenyl]-5-methylpyridin
2-[2-Fluor-4-(2'-trans-4-octylcyclohexylethyl)phenyl]-5-methylpyridin
2-[2-Fluor-4-(2'-trans-4-ethylcyclohexylethyl)phenyl]-5-ethylpyridin
2-[2-Fluor-4-(2'-trans-4-propylcyclohexylethyl)phenyl]-5-ethylpyridin
2-[2-Fluor-4-(2'-trans-4-butylcyclohexylethyl)phenyl]-5-ethylpyridin
2-[2-Fluor-4-(2'-trans-4-pentylcyclohexylethyl)phenyl]-5-ethylpyridin
2-[2-Fluor-4-(2'-trans-4-hexylcyclohexylethyl)phenyl]-5-ethylpyridin
2-[2-Fluor-4-(2'-trans-4-heptylcyclohexylethyl)phenyl]-5-ethylpyridin
2-[2-Fluor-4-(2'-trans-4-octylcyclohexylethyl)phenyl]-5-ethylpyridin
2-[2-Fluor-4-(2'-trans-4-ethylcyclohexylethyl)phenyl]-5-propylpyridin
2-[2-Fluor-4-(2'-trans-4-propylcyclohexylethyl)phenyl]-5-propylpyridin
2-[2-Fluor-4-(2'-trans-4-butylcyclohexylethyl)phenyl]-5-propylpyridin
2-[2-Fluor-4-(2'-trans-4-pentylcyclohexylethyl)phenyl]-5-propylpyridin
2-[2-Fluor-4-(2'-trans-4-hexylcyclohexylethyl)phenyl]-5-propylpyridin
2-[2-Fluor-4-(2'-trans-4-heptylcyclohexylethyl)phenyl]-5-propylpyridin
2-[2-Fluor-4-(2'-trans-4-octylcyclohexylethyl)phenyl]-5-propylpyridin
2-[2-Fluor-4-(2'-trans-4-ethylcyclohexylethyl)phenyl]-5-butylpyridin
2-[2-Fluor-4-(2'-trans-4-propylcyclohexylethyl)phenyl]-5-butylpyridin
2-[2-Fluor-4-(2'-trans-4-butylcyclohexylethyl)phenyl]-5-butylpyridin
2-[2-Fluor-4-(2'-trans-4-pentylcyclohexylethyl)phenyl]-5-butylpyridin
2-[2-Fluor-4-(2'-trans-4-hexylcyclohexylethyl)phenyl]-5-butylpyridin
2-[2-Fluor-4-(2'-trans-4-heptylcyclohexylethyl)phenyl]-5-butylpyridin
2-[2-Fluor-4-(2'-trans-4-octylcyclohexylethyl)phenyl]-5-butylpyridin
2-[2-Fluor-4-(2'-trans-4-ethylcyclohexylethyl)phenyl]-5-pentylpyridin
2-[2-Fluor-4-(2'-trans-4-propylcyclohexylethyl)phenyl]-5-pentylpyridin
2-[2-Fluor-4-(2'-trans-4-butylcyclohexylethyl)phenyl]-5-pentylpyridin
2-[2-Fluor-4-(2'-trans-4-pentylcyclohexylethyl)phenyl]-5-pentylpyridin
2-[2-Fluor-4-(2'-trans-4-hexylcyclohexylethyl)phenyl]-5-pentylpyridin
2-[2-Fluor-4-(2'-trans-4-heptylcyclohexylethyl)phenyl]-5-pentylpyridin
2-[2-Fluor-4-(2'-trans-4-octylcyclohexylethyl)phenyl]-5-pentylpyridin

### Vergleichsbeispiel

In einer Lösung von 14,9 g 5-Butyl-2-picolin in 50 ml Diethylether gibt man unter Eiskühlung 46 ml einer 2,2 molaren Lösung von Phenyllithium in Cyclohexan/Diethylether (7:3).Man rührt 1 Stunde und gibt dann bei Raumtemperatur 15,9 g 4-Ethoxybenzylchlorid in 50 ml Diethylether zu, wobei der Ether zum Sieden kommt. Das Reaktionsgemisch wird nach 1 Stunde auf Eis gegossen und die organische Phase aufgearbeitet. Nach säulenchromatographischer Trennung (Kieselgel, Toluol) und Umkristallisation erhält man 2-(p-Ethoxyphenylethyl)-5-butylpyridin.

Analog erhält man ausgehend von den 5-Picolinen (2-substituiert) und den 1-Alkyl-4-trans-chlormethyl- cyclohexenen folgende Verbindungen:
2-p-Ethylphenyl-5-(trans-4-ethylcyclohexylethyl)-pyridin
2-p-Propylphenyl-5-(trans-4-ethylcyclohexylethyl)-pyridin
2-p-Butylphenyl-5-(trans-4-ethylcyclohexylethyl)-pyridin
2-p-Pentylphenyl-5-(trans-4-ethylcyclohexylethyl)-pyridin
2-p-Hexylphenyl-5-(trans-4-ethylcyclohexylethyl)-pyridin
2-p-Heptylphenyl-5-(trans-4-ethylcyclohexylethyl)-pyridin
2-p-Octylphenyl-5-(trans-4-ethylcyclohexylethyl)-pyridin
2-p-Ethoxyphenyl-5-(trans-4-ethylcyclohexylethyl)-pyridin
2-p-Propoxyphenyl-5-(trans-4-ethylcyclohexylethyl)-pyridin
2-p-Butoxyphenyl-5-(trans-4-ethylcyclohexylethyl)-pyridin
2-p-Pentoxyphenyl-5-(trans-4-ethylcyclohexylethyl)-pyridin
2-p-Hexoxyphenyl-5-(trans-4-ethylcyclohexylethyl)-pyridin
2-p-Heptoxyphenyl-5-(trans-4-ethylcyclohexylethyl)-pyridin
2-p-Octoxyphenyl-5-(trans-4-ethylcyclohexylethyl)-pyridin
2-p-Ethylphenyl-5-(trans-4-propylcyclohexylethyl)-pyridin
2-p-Propylphenyl-5-(trans-4-propylcyclohexylethyl)-pyridin
2-p-Butylphenyl-5-(trans-4-propylcyclohexylethyl)-pyridin
2-p-Pentylphenyl-5-(trans-4-propylcyclohexylethyl)-pyridin
2-p-Hexylphenyl-5-(trans-4-propylcyclohexylethyl)-pyridin
2-p-Heptylphenyl-5-(trans-4-propylcyclohexylethyl)-pyridin
2-p-Octylphenyl-5-(trans-4-propylcyclohexylethyl)-pyridin
2-p-Ethoxyphenyl-5-(trans-4-propylcyclohexylethyl)-pyridin
2-p-Propoxyphenyl-5-(trans-4-propylcyclohexylethyl)-pyridin
2-p-Butoxyphenyl-5-(trans-4-propylcyclohexylethyl)-pyridin
2-p-Pentoxyphenyl-5-(trans-4-propylcyclohexylethyl)-pyridin
2-p-Hexoxyphenyl-5-(trans-4-propylcyclohexylethyl)-pyridin
2-p-Heptoxyphenyl-5-(trans-4-propylcyclohexylethyl)-pyridin
2-p-Octoxyphenyl-5-(trans-4-propylcyclohexylethyl)-pyridin
2-p-Ethylphenyl-5-(trans-4-butylcyclohexylethyl)-pyridin
2-p-Propylphenyl-5-(trans-4-butylcyclohexylethyl)-pyridin
2-p-Butylphenyl-5-(trans-4-butylcyclohexylethyl)-pyridin
2-p-Pentylphenyl-5-(trans-4-butylcyclohexylethyl)-pyridin
2-p-Hexylphenyl-5-(trans-4-butylcyclohexylethyl)-pyridin
2-p-Heptylphenyl-5-(trans-4-butylcyclohexylethyl)-pyridin
2-p-Octylphenyl-5-(trans-4-butylcyclohexylethyl)-pyridin
2-p-Ethoxyphenyl-5-(trans-4-butylcyclohexylethyl)-pyridin
2-p-Propoxyphenyl-5-(trans-4-butylcyclohexylethyl)-pyridin
2-p-Butoxyphenyl-5-(trans-4-butylcyclohexylethyl)-pyridin
2-p-Pentoxyphenyl-5-(trans-4-butylcyclohexylethyl)-pyridin
2-p-Hexoxyphenyl-5-(trans-4-butylcyclohexylethyl)-pyridin
2-p-Heptoxyphenyl-5-(trans-4-butylcyclohexylethyl)-pyridin
2-p-Octoxyphenyl-5-(trans-4-butylcyclohexylethyl)-pyridin
2-p-Ethylphenyl-5-(trans-4-pentylcyclohexylethyl)-pyridin
2-p-Propylphenyl-5-(trans-4-pentylcyclohexylethyl)-pyridin, C 52 SB 133 ° S_{A} 150 •
2-p-Butylphenyl-5-(trans-4-pentylcyclohexylethyl)-pyridin
2-p-Pentylphenyl-5-(trans-4-pentylcyclohexylethyl)-pyridin
2-p-Hexylphenyl-5-(trans-4-pentylcyclohexylethyl)-pyridin
2-p-Heptylphenyl-5-(trans-4-pentylcyclohexylethyl)-pyridin
2-p-Octylohenyl-5-(trans-4-pentylcyclohexylethyl)-pyridin
2-p-Ethoxyphenyl-5-(trans-4-pentylcyclohexylethyl)-pyridin
2-p-Propoxyphenyl-5-(trans-4-pentylcyclohexylethyl)-pyridin
2-p-Butoxyphenyl-5-(trans-4-pentylcyclohexylethyl)-pyridin
2-p-Pentoxyphenyl-5-(trans-4-pentylcyclohexylethyl)-pyridin
2-p-Hexoxyphenyl-5-(trans-4-pentylcyclohexylethyl)-pyridin
2-p-Heptoxyphenyl-5-(trans-4-pentylcyclohexylethyl)-pyridin
2-p-Octoxyphenyl-5-(trans-4-pentylcyclohexylethyl)-pyridin
2-p-Ethylphenyl-5-(trans-4-hexylcyclohexylethyl)-pyridin
2-p-Propylphenyl-5-(trans-4-hexylcyclohexylethyl)-pyridin
2-p-Butylphenyl-5-(trans-4-hexylcyclohexylethyl)-pyridin
2-p-Pentylphenyl-5-(trans-4-hexylcyclohexylethyl)-pyridin
2-p-Hexylphenyl-5-(trans-4-hexylcyclohexylethyl)-pyridin
2-p-Heptylphenyl-5-(trans-4-hexylcyclohexylethyl)-pyridin
2-p-Octylphenyl-5-(trans-4-hexylcyclohexylethyl)-pyridin
2-p-Ethoxyphenyl-5-(trans-4-hexylcyclohexylethyl)-pyridin
2-p-Propoxyphenyl-5-(trans-4-hexylcyclohexylethyl)-pyridin
2-p-Butoxyphenyl-5-(trans-4-hexylcyclohexylethyl)-pyridin
2-p-Pentoxyphenyl-5-(trans-4-hexylcyclohexylethyl)-pyridin
2-p-Hexoxyphenyl-5-(trans-4-hexylcyclohexylethyl)-pyridin
2-p-Heptoxyphenyl-5-(trans-4-hexylcyclohexylethyl)-pyridin
2-p-Octoxyphenyl-5-(trans-4-hexylcyclohexylethyl)-pyridin
2-p-Ethylphenyl-5-(trans-4-heptylcyclohexylethyl)-pyridin
2-p-Propylphenyl-5-(trans-4-heptylcyclohexylethyl)-pyridin
2-p-Butylphenyl-5-(trans-4-heptylcyclohexylethyl)-pyridin
2-p-Pentylphenyl-5-(trans-4-heptylcyclohexylethyl)-pyridin
2-p-Hexylphenyl-5-(trans-4-heptylcyclohexylethyl)-pyridin
2-p-Heptylphenyl-5-(trans-4-heptylcyclohexylethyl)-pyridin
2-p-Octylphenyl-5-(trans-4-heptylcyclohexylethyl)-pyridin
2-p-Ethoxyphenyl-5-(trans-4-heptylcyclohexylethyl)-pyridin
2-p-Propoxyphenyl-5-(trans-4-heptylcyclohexylethyl)-pyridin
2-p-Butoxyphenyl-5-(trans-4-heptylcyclohexylethyl)-pyridin
2-p-Pentoxyphenyl-5-(trans-4-heptylcyclohexylethyl)-pyridin
2-p-Hexoxyphenyl-5-(trans-4-heptylcyclohexylethyl)-pyridin
2-p-Heptoxyphenyl-5-(trans-4-heptylcyclohexylethyl)-pyridin
2-p-Octoxyphenyl-5-(trans-4-heptylcyclohexylethyl)-pyridin
2-p-Ethylphenyl-5-(trans-4-octylcyclohexylethyl)-pyridin
2-p-Propylphenyl-5-(trans-4-octylcyclohexylethyl)-pyridin
2-p-Butylphenyl-5-(trans-4-octylcyclohexylethyl)-pyridin
2-p-Pentylphenyl-5-(trans-4-octylcyclohexylethyl)-pyridin
2-p-Hexylphenyl-5-(trans-4-octylcyclohexylethyl)-pyridin
2-p-Heptylphenyl-5-(trans-4-octylcyclohexylethyl)-pyridin
2-p-Octylphenyl-5-(trans-4-octylcyclohexylethyl)-pyridin
2-p-Ethoxyphenyl-5-(trans-4-octylcyclohexylethyl)-pyridin
2-p-Propoxyphenyl-5-(trans-4-octylcyclohexylethyl)-pyridin
2-p-Butoxyphenyl-5-(trans-4-octylcyclohexylethyl)-pyridin
2-p-Pentoxyphenyl-5-(trans-4-octylcyclohexylethyl)-pyridin
2-p-Hexoxyphenyl-5-(trans-4-octylcyclohexylethyl)-pyridin
2-p-Heptoxyphenyl-5-(trans-4-octylcyclohexylethyl)-pyridin
2-p-Octoxyphenyl-5-(trans-4-octylcyclohexylethyl)-pyridin

Die folgenden Beispiele betreffen erfindungsgemäße flüssigkristalline Phasen:

### Beispiel A:

Eine flüssigkristalline Phase bestehend aus
10 % p-trans-4-Ethylcyclohexylbenzonitril,
15 % p-trans-4-Propylcyclohexylbenzonitril,
15 % p-trans-4-Butylcyclohexylbenzonitril,
20 % p-trans-4-Pentylcyclohexylbenzonitril,
12 % p-trans-4-Hexylcyclohexylbenzonitril,
11 % p-trans-4-Heptylcyclohexylbenzonitril, und
17 % 2-p-[2'-(trans-4-Pentylcyclohexyl)ethyl]-phenyl-5-propylpyridin

hat einen Schmelzpunkt von -11 _{°} und einen Klärpunkt von +62 ° C.

## Patentansprüche

1. Heterocyclische Verbindungen der Formel I 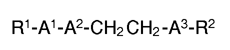 worin
R¹ und R² jeweils unabhängig voneinander eine Alkylgruppe mit 1-15 C-Atomen, worin auch eine oder zwei nicht benachbarte CH₂-Gruppen durch O-Atome und/oder -CO-Gruppen und/oder -O-CO-Gruppen und/oder -CO-O-Gruppen und/oder -O-COO-Gruppen und/oder -CHCN- und/oder -CH-Halogen-Gruppen ersetzt sein können, einer der Reste R¹ und R² auch F, Cl, Br oder CN,
A¹, A² und A³ jeweils unabhängig voneinander eine unsubstituierte oder durch F- und/oder CI-Atome und/oder CH₃- und/oder CN-Gruppen ein- oder mehrfach substituierte 1,4-Cyclohexylengruppe, worin auch eine oder zwei nicht benachbarte CH₂-Gruppen durch O-Atome und/oder S-Atome ersetzt sein können, eine 1,4-Bicyclo-(2,2,2)-octylengruppe oder eine unsubstituierte oder durch F- und/oder CI-Atome und/oder CH₃- und/oder CN-Gruppen ein- oder mehrfach substituierte 1,4-Phenylengruppe, worin auch eine oder mehrere CH-Gruppen durch N ersetzt sein können, bedeutet, mit der Maßgabe , daß
mindestens eine der Gruppen A¹ und A² eine Pyrazin-2,5-diylgruppe (Pa) oder eine Pyridin-2,5-diylgruppe (Py) ist,

2. Heterocyclische Verbindungen nach Anspruch 1, dadurch gekennzeichnet, daß A¹ und A² jeweils unabhängig voneinander eine trans- 1,4-Cyclohexylengruppe, eine 1,4-Phenylengruppe, die gegebenenfalls auch durch F substituiert sein kann, eine Pyridin 2,5-diylgruppe oder eine Pyrazin-2,5- diylgruppe und A³ eine trans-1,4-Cyclohexylengruppe oder eine 1,4-Phenylengruppe bedeutet, die gegebenenfalls auch durch F substituiert sein kann.

3. Heterocyclische Verbindungen nach Anspruch 1 oder 2, dadurch gekennzeichnet, daß einer der Reste R¹ und R² F, CI oder Br bedeutet.

4. Heterocyclische Verbindungen nach Anspruch 3, dadurch gekennzeichnet, daß einer der Reste R¹ und R² Alkyl und der andere F bedeutet.

5. Heterocyclische Verbindungen nach Anspruch 1 oder 2, dadurch gekennzeichnet, daß einer der Reste A¹, A² und A³ eine durch F-Atome substituierte 1,4-Phenylengruppe ist.

6. Heterocyclische Verbindungen nach Anspruch 1 oder 2, dadurch gekennzeichnet, daß einer der Reste A¹ und A² eine Pyrazin-2,5-diylgruppe ist.

7. Heterocyclische Verbindungen nach Anspruch 1 oder 2, dadurch gekennzeichnet, daß einer der Reste A¹ und A² eine Pyridin-2,5-diylgruppe ist und die Verbindungen mindestens eine lateral substituierte 1,4-Phenylengruppe enthalten.

8. Heterocyclische Verbindungen nach Anspruch 7, dadurch gekennzeichnet, daß die lateral substituierte 1,4-Phenylengruppe 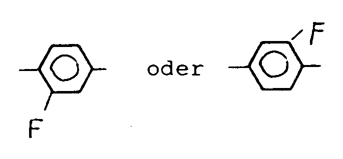 bedeutet.

9. Verfahren zur Herstellung von heterocyclischen Verbindungen der Formel 1 nach Anspruch 1, dadurch gekennzeichnet, daß man eine Verbindung, die sonst der Formel I entspricht, aber an Stelle von H-Atomen eine oder mehrere reduzierbare Gruppen und/oder C-C-Bindungen enthält, mit einem reduzierenden Mittel behandelt, oder daß man zur Herstellung von Estern der Formel I (worin R¹ und/oder R² eine Alkylgruppe bedeuten, worin eine oder zwei CH₂-Gruppen durch -O-CO-Gruppen und/oder -CO-O-Gruppen ersetzt sind)
eine entsprechende Carbonsäure oder eines ihrer reaktionsfähigen Derivate mit einem entsprechenden Alkohol oder einem seiner reaktionsfähigen Derivate umsetzt,
oder daß man zur Herstellung von 1,3-Dioxanderivaten bzw. 1,3-Dithianderivaten der Formel I (worin A¹ oder A² oder A³ 1,3-Dioxan-2,5-diyl bzw. 1,3-Dithian-2,5-diyl bedeutet) einen entsprechenden Aldehyd mit einem entsprechenden Diol bzw. Dithiol umsetzt,
oder daß man zur Herstellung von Ethern der Formel I (worin R¹ und/oder R² eine Alkylgruppe bedeutet, worin eine oder zwei CH₂-Gruppen durch O-Atome ersetzt sind)
eine entsprechende Hydroxyverbindung verethert,
oder daß man zur Herstellung von Nitrilen der Formel I (worin R¹ oder R² CN bedeutet und/oder A¹ und/oder A² und/oder A³ durch mindestens eine CN-Gruppe substituiert ist) die entsprechenden Chlor-oder Bromverbindungen mit einem Cyanid umsetzt, und/oder daß man gegebenenfalls eine Base der Formel I durch Behandeln mit einer Säure in eines ihrer Säureadditionssalze umwandelt,
oder daß man gegebenenfalls eine Verbindung der Formel I aus einem ihrer Säureadditionssalze durch Behandeln mit einer Base freisetzt,
oder daß man zur Herstellung von Nitrilen der Formel I (worin R¹ oder R² CN bedeutet und/oder worin A¹ und/oder A² und/oder A³ durch mindestens eine CN-Gruppe substituiert sind) entsprechende Säureamide dehydratisiert oder die entsprechenden Säurehalogenide mit Sulfamid umsetzt,
oder daß man zur Herstellung von Verbindungen der Formel I (worin A¹ und/oder A² und/oder A³ Aromaten bedeuten und durch F- und/oder CI-Atome substituiert sind) die entsprechenden Diazoniumtetrafluoroborate thermisch zersetzt oder zur Einführung des CI-Atoms die entsprechenden Diazoniumsalze mit Kupfer-(I)-chlorid behandelt.

10. Verwendung der Verbindungen der Formel 1 nach Anspruch 1 als Komponenten flüssigkristalliner Phasen.

11. Flüssigkristalline Phase mit mindestens zwei flüssigkristallinen Komponenten, dadurch gekennzeichnet, daß mindestens eine Komponente eine Verbindung der Formel 1 ist.

12. Elektrooptisches Anzeigeelement, dadurch gekennzeichnet, daß es als Dielektrikum eine Phase nach Anspruch 11 enthält.

## Claims

1. Heterocyclic compounds of the formula I 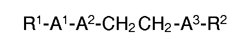 wherein
R¹ and R² are each independently of the other an alkyl group of 1-15 C atoms, wherein one or two non-adjacent CH₂ groups can also be replaced by O atoms and/or -CO-groups and/or -O-CO- groups and/or -CO-O- groups and/or -O-COO- groups and/or -CHCN- and/or -CH-halogen groups, and one of the radicals R¹ and R² is also F, Cl, Br or CN,
A¹, A² and A3 are each independently of the other a 1,4-cyclohexylene group which is unsubstituted or monosubstituted or polysubstituted by F and/or CI atoms and/or CH₃ and/or CN groups and wherein one or two non-adjacent CH₂ groups can be replaced by O atoms and/or S atoms, or are a 1,4-bicyclo(2,2,2)octylene group, or a 1,4-phenylene group which is unsubstituted or monosubstituted or polysubstituted by F and/or CI atoms and/or CH₃ and/or CN groups and wherein one or more CH groups can be replaced by N,
with the proviso that at least one of the groups A¹ and A² is a pyrazine-2,5-diyl group (Pa) or a pyridine-2,5-diyl group (Py).

2. Heterocyclic compounds according to Claim 1, characterized in that A¹ and A² are each independently of the other a trans-1,4-cyclohexylene group, a 1,4-phenylene group which can optionally also be substituted by F, a pyridine-2,5-diyl group or a pyrazine-2,5-diyl group, and A3 is a trans-1,4-cyclohexylene group or a 1,4-phenylene group which can optionally also be substituted by F.

3. Heterocyclic compounds according to Claim 1 or 2, characterized in that one of the radicals R¹ and R² is F, CI or Br.

4. Heterocyclic compounds according to Claim 3, characterized in that one of the radicals R¹ and R² is alkyl and the other is F.

5. Heterocyclic compounds according to Claim 1 or 2, characterized in that one of the radicals A¹, A² and A3 is a 1,4-phenylene group which is substituted by F atoms.

6. Heterocyclic compounds according to Claim 1 or 2, characterized in that one of the radicals A¹ and A² is a pyrazine-2,5-diyl group.

7. Heterocyclic compounds according to Claim 1 or 2, characterized in that one of the radicals A¹ and A² is a pyridine-2,5-diyl group, and the compounds contain at least one laterally substituted 1,4-phenylene group.

8. Heterocyclic compounds according to Claim 7, characterized in that the laterally substituted 1,4-phenylene group is

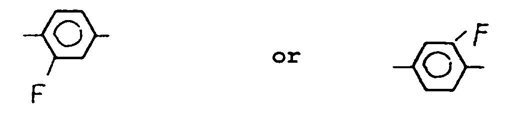

9. Process for the preparation of heterocyclic compounds of the formula I according to Claim 1, characterized in that a compound which conforms to the formula I, but contains one or more reducible groups and/or C-C bonds in place of H atoms is treated with a reducing agent,
or in that, to prepare esters of the formula I (wherein R¹ and/or R² are an alkyl group wherein one or two CH₂ groups have been replaced by -O-CO-groups and/or -CO-O-groups), a corresponding carboxylic acid or a reactive derivative thereof is reacted with an appropriate alcohol or a reactive derivative thereof,
or in that, to prepare 1,3-dioxane derivatives or 1,3-dithiane derivatives of the formula I (wherein A¹ or A² or A3 is 1,3-dioxane-2,5-diyl or 1,3-dithiane-2,5-diyl), a corresponding aldehyde is reacted with an appropriate diol or dithiol,
or in that, to prepare ethers of the formula I (wherein R¹ and/or R² is an alkyl group wherein one or two CH₂ groups have been replaced by O atoms), a corresponding hydroxyl compound is etherified, or in that, to prepare nitriles of the formula I (wherein R¹ or R² is CN and/or A¹ and/or A² and/or A3 is substituted by at least one CN group), the corresponding chlorine or bromine compounds are reacted with a cyanide and/or in that a base of the formula I is optionally converted into an acid-addition salt thereof by treatment with an acid,
or in that a compound of the formula I is optionally liberated from an acid-addition salt thereof by treatment with a base,
or in that, to prepare nitriles of the formula I (wherein R¹ or R² is CN and/or wherein A¹ and/or A² and/or A3 are substituted by at least one CN group), corresponding acid amides are dehydrated or the corresponding acid halides are reacted with sulphamide,
or in that, to prepare compounds of the formula I (in which A¹ and/or A² and/or A3 are aromatics and are substituted by F and/or CI atoms), the corresponding diazonium tetrafluoroborates are decomposed thermally or, to introduce the CI atoms, the corresponding diazonium salts are treated with copper(l) chloride.

10. Use of the compounds of the formula I according to Claim 1 as components of liquid-crystalline phases.

11. Liquid-crystalline phase containing at least two liquid-crystalline components, characterized in that at least one component is a compound of the formula I.

12. Electrooptical display element, characterized in that it contains as dielectric a phase according to Claim 11.

## Revendications

1. Composés hétérocycliques de formule 1 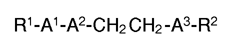 dans laquelle
R¹ et R² chaque fois indépendamment l'un de l'autre représentent un groupe alkyle ayant de 1 à 15 atomes de C, où aussi un ou deux groupes CH₂ non voisins peuvent être remplacés par des atomes de O et/ou des groupes -CO- et/ou des groupes -O-CO- et/ou des groupes -CO-O- et/ou des groupes -O-COO- et/ou des groupes -CHCN et/ou des groupes -CH-halogène, un des radicaux R¹ et R² représentant aussi F, CI, Br ou CN,
A¹, A² et A³ chaque fois indépendamment les uns des autres représentent un groupe 1,4-cyclohexylène non substitué ou substitué une ou plusieurs fois par des atomes de F et/ou CI et/ou des groupes CH₃ et/ou CN, où aussi un ou deux groupes CH₂ non voisins peuvent être remplacés par des atomes de O et/ou des atomes de S, un groupe 1,4-bicyclo-(2,2,2)-octylène ou un groupe 1,4-phénylène non substitué ou substitué une ou plusieurs fois par des atomes de F et/ou CI et/ou des groupes CH₃ et/ou CN, où aussi un ou plusieurs groupes CH peuvent être remplacés par N,
à la condition qu'au moins un des groupes A¹ et A² soit un groupe pyrazine-2,5-diyle (Pa) ou un groupe pyridine-2,5-diyle (Py).

2. Composés hétérocycliques selon la revendication 1, caractérisés en ce que A¹ et A², chaque fois indépendants l'un de l'autre, représentent un groupe trans-1,4-cyclohexylène, un groupe 1,4-phénylène, qui peut aussi être substitué par F, un groupe pyridine-2,5-diyle ou un groupe pyrazine-2,5-diyle et A³ représente un groupe trans-1,4-cyclohexylène ou un groupe 1,4-phénylène, qui peut aussi être substitué le cas échéant par F.

3. Composés hétérocycliques selon la revendication 1 ou 2, caractérisés en ce que l'un des radicaux R¹ et R² représente F, CI ou Br.

4. Composés hétérocycliques selon la revendication 3, caractérisés en ce que l'un des radicaux R¹ et R² représente un alkyle et l'autre représente F.

5. Composés hétérocycliques selon la revendication 1 ou 2, caractérisés en ce que l'un des radicaux A¹, A² et A³ est un groupe 1,4-phénylène substitué par un atome de F.

6. Composés hétérocycliques selon la revendication 1 ou 2, caractérisés en ce que l'un des radicaux A¹ et A² est un groupe pyrazine-2,5-diyle.

7. Composés hétérocycliques selon la revendication 1 ou 2, caractérisés en ce que l'un des radicaux A¹ et A² est un groupe pyridine-2,5-diyle et les composés comportent au moins un groupe 1,4-phényléne à substitution latérale.

8. Composés hétérocycliques selon la revendication 7, caractérisés en ce que le groupe 1,4-phénylène à substitution latérale représente

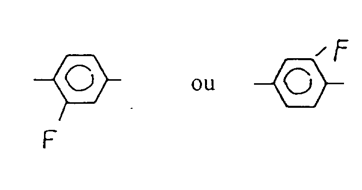

9. Procédé pour préparer les composés hétérocycliques de formule I selon la revendication 1, caractérisé en ce que l'on traite avec un agent réducteur un composé qui correspond à la formule I avec la différence qu'au lieu d'atomes de H il comporte un ou plusieurs groupes que l'on peut réduire et/ou une ou plusieurs liaisons C-C,
ou caractérisé en ce que, pour préparer des esters de formule 1 (où R¹ et/ou R² représentent un groupe alkyle, où un ou deux des groupes CH₂ sont remplacés par des groupes O-CO- et/ou des groupes -CO-O-) on fait réagir un acide carboxylique approprié, ou un de ses dérivés susceptibles de réagir, avec un alcool approprié, ou un de ses dérivés susceptibles de réagir,
ou caractérisé en ce que, pour préparer des dérivés du 1,3-dioxanne ou des dérivés du 1,3-dithianne de formule 1 (où A¹ ou A² ou A³ représente le 1,3-dioxanne-2,5-diyle ou le 1,3-dithianne-2,5-diyle), on fait réagir un aldéhyde approprié avec un diol ou un dithiol approprié,
ou caractérisé en ce que, pour préparer des éthers de formule 1 (où R¹ et/ou R² représentent un groupe alkyle, où un ou deux des groupes CH₂ sont remplacés par des atomes de O) on éthérifie un composé hydroxy approprié,
ou caractérisé en ce que, pour préparer des nitriles de formule 1 (où R¹ ou R² représente CN, et/ou A¹ et/ou A² et/ou A³ sont substitués par au moins un groupe CN), on fait réagir les composés chlorés ou bromés appropriés avec un cyanure,
et/ou caractérisé en ce que l'on transforme éventuellement une base de formule I, par traitement avec un acide, en un de ses sels d'addition d'acide,
ou caractérisé en ce que, éventuellement, on libère un composé de formule I à partir d'un de ses sels d'addition d'acide par traitement avec une base,
ou caractérisé en ce que, pour préparer des nitriles de formule 1 (où R¹ ou R² représente CN, et/ou A¹ et/ou A² et/ou A³ sont substitués par au moins un groupe CN), on déshydrate les amides d'acides appropriés, ou on fait réagir les halogénures d'acides appropriés avec un sulfamide,
ou caractérisé en ce que, pour préparer les composés de formule 1 (où A¹ et/ou A² et/ou A³ représentent des aromatiques et sont substitués par des atomes de F et/ou CI,) on décompose thermiquement les tétrafluoroborates de diazonium appropriés ou, pour introduire l'atome de CI, on traite les sels appropriés de diazonium avec le chlorure de cuivre (1).

10. Utilisation des composés de formule I selon la revendication 1 comme composants de phases à cristaux liquides.

11. Phase à cristaux liquides comportant au moins deux composants à cristaux liquides, caractérisée en ce qu'au moins un composant est un composé de formule I.

12. Elément d'affichage électro-optique, caractérisé en ce qu'il comporte comme diélectrique une phase selon la revendication 11.
